# EUROPEAN PATENT APPLICATION

(11) **EP 1 557 151 A1**
(43) Date of publication of application: **27.07.2005**
(21) Application number: 04001328.6
(22) Date of filing: 22.01.2004
(51) Int. Cl.: A61J 3/00, A61K 9/20

(54) **Solid dosage form**

(71) Applicant: F. HOFFMANN-LA ROCHE AG, 4070 Basel (CH)
(72) Inventor: Rothenhaeusler, Benno, 79541 Loerrach (DE); Alex, Rainer, 79576 Weil am Rhein (DE)
(74) Representative: Heiroth, Ulrike, Dr.

(57) **Abstract**

A solid dosage form (1) comprising a predefined magnetic pattern (2) representing information. The dosage form (1) may be an oral dosage form such as a tablet or a capsule, and the magnetic pattern (2) may be invisible. The information contained in the magnetic pattern (2) may be coded.

## Description

The present invention deals with a solid dosage form according to the independent patent claim.

Solid dosage forms are conventional dosage forms that are well accepted around the whole globe. While the term "solid dosage form" within this specification is intended to cover all types of solid dosage forms (e.g. tablets, capsules suppositories, etc.) and all types of applications (e.g. cosmetic, pharmaceutical, diagnostic, nutritional, dietary etc. applications), solid dosage forms are of particular interest as oral dosage forms containing a pharmaceutically active ingredient, a cosmetic ingredient, a diagnostic reagent, a nutritional or dietary supplement, etc..

Especially in the field of medicaments counterfeit protection is getting more and more an important feature, since regulatory authorities in charge of admission of medicaments to the respective domestic markets are putting more and more burden on manufacturers that their medicaments must have some protection against counterfeiting, since - depending on the country - considerable amounts of counterfeited illegal medicaments have been found to have penetrated into the market. Especially in the case of life-supporting medicaments this could have severe consequences for patients.

While the imprinting of logos of the manufacturer company into the outer surface of a tablet is a conventional measure to identify the origin of the tablet, the level of protection against counterfeiting provided by this measure is only low. On the other hand, suppliers (such as pharmacies, physicians, etc.) want to be sure or need to be sure (e.g. for liability reasons), that it is the original product from a specific manufacturer at the time they distribute it to consumers.

Also, the identity of a solid dosage form must be ensured and documented throughout the whole manufacturing process and during storage and distribution. This is usually performed in a non-destructive way by assessing some characteristic visible features of the dosage form like imprint, color or shape. These features could either be assessed by a human or read by a machine. However, it is at least difficult if not impossible to assess or read these features without establishing mechanical contact between the dosage form and the reader except when the dosage form is exposed to a suitable external source of light or radiation. Also, quality control cannot easily be performed.

Accordingly, it is an object of the invention to suggest a solid dosage form that overcomes the aforementioned disadvantages.

This object is achieved with the solid dosage form according to the invention as characterized by the features of the independent patent claim. Advantageous embodiments of the solid dosage form become apparent from the features of the dependent patent claims.

In particular, the solid dosage form according to the invention comprises a predefined magnetic pattern representing information. A magnetic pattern may not be easily detected by potential counterfeiters, and even in case it is detected by a counterfeiter it is difficult to be copied. On the other hand, a suitable magnetic pattern is comparatively easy to manufacture. Also, with a suitable means the suppliers (e.g. pharmacies, physicians, etc.) may easily verify that it is the original product that they distribute to consumers. Also, the magnetic pattern allows quality control as well as assessment of the identity of the dosage form without a need for external sources of light or radiation.

In particular, the magnetic pattern of the solid dosage form according to the invention is provided by a physiologically acceptable magnetized ancillary substance. The physiologically acceptable magnetized ancillary substance ensures that the magnetic pattern is not detrimental to the consumer.

The solid dosage form may be a pharmaceutically active, cosmetic, diagnostic, nutritional, dietary, etc., dosage form, in particular an oral dosage form (such as a tablet, e.g. coated tablet, multi-layer tablet, dragée, pill, granules, powder, or capsule) containing a pharmaceutically active ingredient, a cosmetic ingredient, a diagnostic reagent, a nutritional or dietary supplement, etc. (this is meant to also include any combination of such ingredients). These types of dosage forms are widespread and well accepted by consumers.

The information contained in the magnetic pattern is advantageously undetectable for a human without any separate detection (e.g. visualization) means. For example, the magnetic pattern can be provided in the interior of the dosage form so that a potential counterfeiter is *prima facie* unable to recognize that there is any protection against counterfeiting at all contained in the dosage form.

While the information may be contained in any form, for example it can be a simple representation of the manufacturer's logo, it may be more advantageous if the information contained in the magnetic pattern is encoded. Encoded information raises the level of protection against counterfeiting, since the counterfeiter may not easily decode the information contained in the code and thus counterfeiting becomes even more difficult.

In particular, the physiologically acceptable magnetized ancillary substance is selected from iron (Fe), iron-11-oxide (Fe₂O₃) or iron-III-oxide (Fe₃O₄). These ancillary substances are known as being physiologically acceptable and magnetizable so as to form the magnetic pattern.

The solid dosage form may comprise a core carrying the magnetic pattern and at least one coating covering the core. In particular, the coating covering the core may be opaque, so that the magnetic pattern carried by the core is undetectable (e.g. invisible) for a human without any specific detection (e.g. visualization) means.

Alternatively, the coating may carry the magnetic pattern rather than the core. Also in this case, the magnetic pattern may be detectable or undetectable for a human. If necessary, a further coating may be provided which may be opaque in order to ensure that the magnetic pattern is undetectable (e.g. invisible).

In one embodiment the solid dosage form may also comprise a separately manufactured film affixed to the dosage form, the film carrying the magnetic pattern. This embodiment allows to separately manufacture both the raw dosage form and the film carrying the magnetic pattern. The film is then affixed to the raw dosage form to form the final dosage form. For example, the film may be a polymer film that is separately manufactured and then attached to the raw dosage form. Again, the magnetic pattern contained in the film may be detectable (e.g. visible) or may be undetectable (e.g.invisible) for a human (e.g. it may be visible only with a specific visualization means).

Further advantageous features of the invention will become apparent from the following detailed description of an embodiment of the invention with the aid of the drawings, in which:
- Fig. 1: shows an embodiment of a solid dosage form carrying a magnetic pattern representing information, in accordance with the instant invention,
- Fig. 2: shows an embodiment of an arrangement for producing the magnetic pattern contained in the embodiment of the dosage form of Fig. 1.

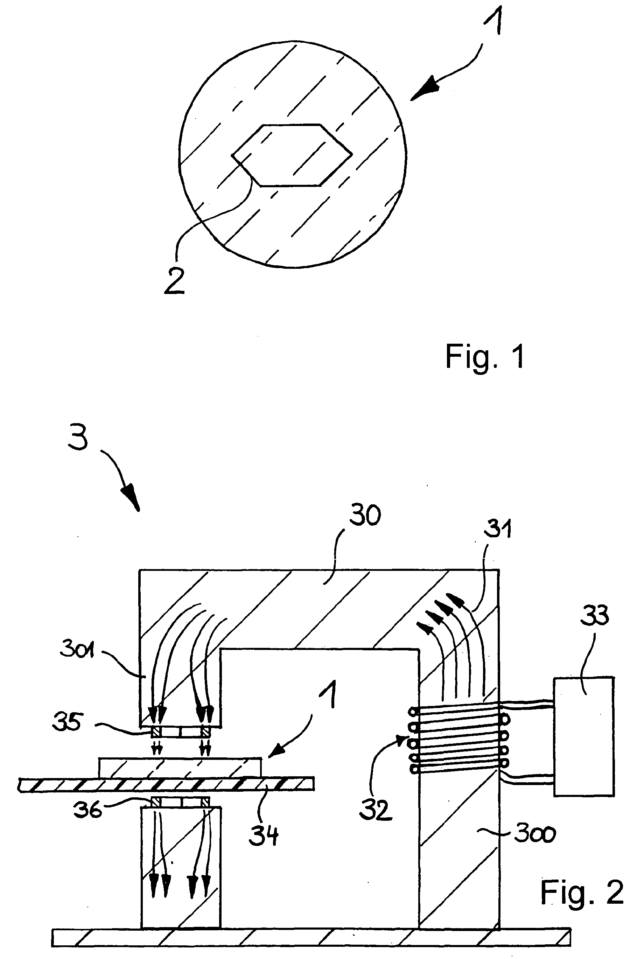

**Fig. 1** shows an embodiment of a solid dosage form according to the instant invention in the form of a tablet 1 comprising a pharmaceutically active ingredient, although the tablet does not mandatorily contain a pharmaceutically active ingredient but instead may contain other active ingredients, e.g. vitamins or ingredients for obtaining cosmetic effects, diagnostic ingredients, nutritional or dietary ingredients, etc., or may contain combinations of different types of active ingredients. Tablet 1 comprises a magnetic pattern 2. Magnetic pattern 2 has the form of a specific hexagon which may constitute a part of the logo of a specific manufacturer (such as applicant's logo). A logo helps to identify the manufacturer of tablet 1 but does not represent a sophisticated code. Therefore, as already described hereinbefore, magnetic pattern 2 may appear in the form of a more sophisticated code, such as a bar code. In this case, the information (e.g. an information about the manufacturer, the name of the medicament, a production lot number, the production date, etc.) may be included in the specific bar code and it is considerably more difficult for counterfeiters - if possible at all - to exactly copy the bar code. As a further alternative, the logo may comprise different precisely located points on the logo which generate a magnetic field that is considerably stronger than the field generated by the remainder of points located on the logo. At these precisely located points, the magnetic field is stronger than a predefined threshold value. Such a specific code is practically impossible to be detected by a counterfeiter, but during verification of the final product to be distributed to the consumer the suppliers (e.g. pharmacies) may easily verify that it is the original product that they distribute to consumers rather than a counterfeit, for example with the aid of a suitable device (detecting the magnetic pattern and comparing the field strength at the precisely located points with the threshold value).

As is illustrated in Fig. 1 by the hatching, magnetic pattern 2 may be invisible (i.e. undetectable) for a human from outside tablet 1 without the use of a specific visualization (i.e. detection) means. In order to achieve this, magnetic pattern 2 may be contained in the core of tablet 1. The core may be covered by at least one opaque coating (not shown in Fig. 1) in order to render magnetic pattern 2 invisible.

Alternatively, magnetic pattern 2 may be contained in the coating. The coating may be opaque so that magnetic pattern 2 is invisible for a human. In case the coating containing magnetic pattern 2 is not opaque the coating itself may be covered by an additional opaque coating in order to ensure that magnetic pattern 2 is invisible.

Furthermore, magnetic pattern 2 may be contained in a film that can be separately manufactured, e.g. a polymer film (e.g. a polymer film containing a magnetizable ancillary substance), which is then affixed (e.g. bonded) to the raw tablet in order to form the final tablet 1.

Magnetic pattern 2 can be provided within tablet 1 by at least one physiologically acceptable magnetized anciallary substance, such as for example iron (Fe), iron-11-oxide (Fe₂O₃) or iron-III-oxide (Fe₃O₄). These ancillary substances are known as being physiologically acceptable.

Magnetic pattern 2 can also be produced by printing the pattern onto the core or onto the coating using a magnetizable, physiologically acceptable ink, and then drying the ink. The ink can be applied in a magnetic field so that the magnetizable particles contained in the ink are immediately magnetized, or the ink can be applied and dried and then magnetization is performed, for example with the aid of a movable writing head generating a suitable magnetic field. Also, a visible (non-magnetic) pattern may be provided carrying an overlaid invisible magnetic pattern.

One embodiment of an arrangement 3 for producing the magnetic pattern contained in tablet 1 shown in Fig. 1 is represented in **Fig. 2**. Arrangement 3 comprises an iron core 30 for guiding a magnetic flux 31 that is generated by a winding 32 arranged on a leg 300 of iron core 30. Winding 32 is supplied with an electric current by a power supply 33 to which it is connected. The electric current flowing through winding 32 produces magnetic flux 31 represented by the respective arrows shown in Fig. 2. In an air gap provided in a further leg 301 of iron core 30 there is arranged a non-magnetic carrier 34 that carries tablet 1. The dimensions of the air gap are represented highly exaggerated in Fig. 2 for clearness reasons. For the sake of simplicity let us assume, that a magnetizable ancillary substance is already provided in tablet 1 but has not yet been magnetized. Upon switching on power supply 33, magnetic flux 31 is generated and guided through iron core 30. On both sides of tablet 1 or carrier 34, respectively, there are arranged templates 35 and 36 corresponding to the shape of magnetic pattern 2 shown in Fig. 1. Since templates 35 and 36 are made from iron (or a material having a high magnetic permeability µ when compared to air) essentially the whole magnetic flux is guided through templates 35 and 36. As a consequence, the magnetizable ancillary substance is magnetized to form magnetic pattern 2 having the shape as shown in Fig. 1.

As has been described above, instead of providing fixedly arranged templates 35 and 36, a moveable writing head can be moved along the contour of the logo so as to produce magnetic pattern 2. Also, other methods (see further above) may be applied to generate magnetic pattern 2.

Magnetic pattern 2 contained in tablet 1 can be detected by any suitable means. Recently, thin transparent magneto-optical films have become available for the direct visualization of magnetic fields (e.g. a film offered under the trade name Kel-View™, available from Kelvin, Inc., USA).

Other detection systems are also contemplated which are based on the principle of electromagnetic induction. For example, when the tablet is moved relative to a sensor of the detection system, the "magnet" (the tablet) in motion produces a time varying magnetic field that induces an electric current in the sensor. Sensors of this type are already used, for example, in tape recorders or card readers. Other types of detection systems utilize the so-called "Hall-effect", that is to say an electric current passing through a conductor is modified by a static magnetic field (the magnetic field acts upon the moving electrons in the conductor). Such detection systems are already used, for example, for counterfeit detection of checks. Further types of detection systems comprise hand-held pens for reading out magnetic information (e.g. of bar codes etc.). Such detection systems are commercially available from a plurality of manufacturers, for example from Stopfraud, Inc., Atlanta, USA). The electric signals produced by the aforedescribed detection systems may be converted so that a respective image can be produced on a conventional screen.

As can be seen, the solid dosage form according to the invention offers a measure (providing a magnetic pattern) that is simple to manufacture but difficult to counterfeit. While the embodiments described above are intended to show examples of the invention, the scope of protection is intended to be defined by the appended claims.

## Claims

1. Solid dosage form (1) comprising a predefined magnetic pattern (2) representing information.

2. Solid dosage form (1) according to claim 1, wherein the magnetic pattern (2) is provided by at least one physiologically acceptable magnetized ancillary substance.

3. Solid dosage form (1) according to any one of the preceding claims, wherein the solid dosage form (1) is a pharmaceutically active, cosmetic, diagnostic or dietary dosage form.

4. Solid dosage form (1) according to any one of the preceding claims, wherein the solid dosage form (1) is an oral dosage form.

5. Solid dosage form (1) according to claim 4, wherein the oral dosage form is a tablet or a capsule.

6. Solid dosage form (1) according to any one of the preceding claims, wherein the information contained in the magnetic pattern (2) is undetectable for a human without any separate detection means.

7. Solid dosage form (1) according to any one of the preceding claims, wherein the information contained in the magnetic pattern (2) is encoded.

8. Solid dosage form (1) according to any one of claims 2 to 7, wherein the physiologically acceptable magnetized ancillary substance is selected from iron (Fe), iron-II-oxide (Fe₂O₃) or iron-III-oxide (Fe₃O₄).

9. Solid dosage form (1) according to any one of the preceding claims, wherein the dosage form (1) comprises a core carrying the magnetic pattern (2) and at least one coating covering the core.

10. Solid dosage form (1) according to any one of the preceding claims, wherein the coating covering the core is opaque.

11. Solid dosage form (1) according to any one of claims 1 to 8, wherein the dosage form comprises a core and at least one coating covering the core, the coating carrying the magnetic pattern (2).

12. Solid dosage form (1) according to any one of claims 1 to 8, comprising a separately manufactured film affixed to the dosage form, the film carrying the magnetic pattern (2).
